# EUROPEAN PATENT SPECIFICATION

(11) **EP 3 530 221 B1**
(45) Date of publication and mention of the grant of the patent: **16.12.2020**
(21) Application number: 19159208.8
(22) Date of filing: 25.02.2019
(51) Int. Cl.: A61B 17/34, A61B 34/20

(54) **SYSTEM FOR PERFORMING A PERCUTANEOUS NAVIGATION PROCEDURE**
SYSTEM ZUR DURCHFÜHRUNG EINES VORGANGS MIT PERKUTANER NAVIGATION
SYSTÈME POUR LA RÉALISATION D'UNE PROCÉDURE DE NAVIGATION PERCUTANÉE

(30) Priority: 26.02.2018 US 201815904744
(43) Date of publication of application: 28.08.2019
(73) Proprietor: Covidien LP, Mansfield, MA 02048 (US)
(72) Inventor: KRIMSKY, William, S, Forest Hill, MD 21050 (US); STOPEK, Joshua, B, Minneapolis, MN 55419 (US)
(74) Representative: Maschio, Antonio

(56) References cited:
- WO-A1-2017/059860
- WO-A2-2009/109879
- US-A1- 2008 033 450
- US-A1- 2016 128 669

## Description

### BACKGROUND

### Technical Field

The present disclosure relates to systems for planning and performing a percutaneous navigation procedure or surgery, and more particularly, to systems for performing a percutaneous navigation procedure or surgery using a trackable needle assembly as a port.

### Description of Related Art

Ultrasound scans of a patient's body are commonly used to confirm placement of surgical tools at treatment locations inside the patient's body. However, once treatment or surgery commences, there exists no way to predict or confirm placement of surgical tools in relation to the treatment locations inside the patient's body. The present disclosure provides systems that provide a user the ability to confirm/visualize the placement of surgical tools in relation to the treatment locations inside the patient's body.

US2016/128669A describes a tracking system that includes a treatment probe including a first tracking sensor configured to track a location of the treatment probe, an ultrasound imager including a second tracking sensor configured to track a location of the ultrasound imager, and a tracking system. The tracking system receives location information from the first and second tracking sensors, tracks the location of the treatment probe and the location of the ultrasound imager, and displays the real-time ultrasound images and a representation of the treatment probe in one or more pre-stored images.

WO2017/059860A describes a depiction system for generating a real time correlated depiction of movements of a surgical tool for uses in minimally invasive surgery. The system comprises computer system, 3D surface data generation means, and position data generation means for obtaining real time spatial position data of at least a part of the surgical tool.

WO2009/109879A describes a system for providing integrated guidance for positioning a needle in a body. The system comprises a non-invasive tracking system for tracing the biopsy device in the body, for providing coarse guidance. The system further comprises an optical detector mounted on the needle for obtaining optical information discriminating tissue in the body, for providing fine guidance.

### SUMMARY

The present invention provides a system as defined in claim 1 for performing a percutaneous navigation procedure. The system includes a trackable needle assembly including a first tracking sensor disposed thereon and defining a lumen therethrough; a camera attached to the trackable needle assembly, the camera configured to capture video or images; a surgical instrument including a second tracking sensor disposed thereon, the surgical instrument configured to be inserted through the lumen of the trackable needle assembly; an ultrasound sensor including a tracking element disposed thereon; and a computing device configured to: track a location of each of the ultrasound sensor, the trackable needle assembly, and the surgical instrument; and display the location of each of the ultrasound sensor, the trackable needle assembly, and the surgical instrument in relation to one another.

In an aspect of the present invention, the computer device is configured to receive video or images captured by the camera and display the received video or images. The displayed video or images of the camera is continuously updated as the trackable needle assembly is navigated. In an aspect of the present invention the camera of the trackable needle assembly is integrally formed with the trackable needle assembly. In another aspect of the invention, the camera of the trackable needle assembly is detachable connected to the trackable needle assembly. The trackable needle assembly includes a plurality of camera guiding features configured to connect the camera to the trackable needle assembly.

In another aspect of the present invention, an electromagnetic field generator is configured to generate an electromagnetic field to be sensed by the sensor of the trackable needle assembly, the sensor of the surgical instrument, and the tracking element of the ultrasound sensor.

### BRIEF DESCRIPTION OF THE DRAWINGS

Objects and features of the presently disclosed system will become apparent to those of ordinary skill in the art when descriptions of various embodiments thereof are read with reference to the accompanying drawings, of which:
FIG. 1 is a perspective view of a system for performing a percutaneous navigation procedure including a trackable needle assembly in accordance with the present invention
FIG. 2A is a perspective view of the trackable needle assembly in accordance with one aspect of the present invention
FIG. 2B is a perspective view of the trackable needle assembly in accordance with another aspect of the present invention
FIG. 2C is a cross-section of the trackable needle assembly in FIG. 2B as taken along section 2C-2C shown in FIG. 2B;
FIG. 3 is a schematic diagram of a computing device which forms part of the system of FIG. 1 in accordance with the present disclosure;
FIG. 4 is an illustration of a user interface presenting a view showing a setup step of the procedure phase of the percutaneous navigation procedure in accordance with the present disclosure;
FIG. 5 is an illustration of a user interface presenting a view showing a guidance step of the procedure phase of the percutaneous navigation procedure in accordance with the present disclosure;
FIG. 6A is a flow chart illustrating a method for planning and performing a percutaneous navigation procedure including a treatment planning phase and a treatment procedure phase in accordance with the present disclosure;
FIG. 6B is a flow chart illustrating a method for planning and performing a percutaneous navigation procedure including a treatment planning phase and a treatment procedure phase in accordance with the present disclosure;
FIG. 7 is an illustration of a user interface presenting a view for reviewing a 3D model of the treatment plan in accordance with the present disclosure;
FIG. 8 is an illustration of the user interface of FIG. 7 illustrating a representation of a patient's skin rendered over the 3D model;
FIG. 9 is an illustration of a user interface presenting a view illustrating a representation of a patient's lung rendered in a 3D model and including a representation of a trackable needle assembly positioned along an access route in accordance with an embodiment of the present disclosure;
FIG. 10 is a flow chart illustrating a method of treatment, including planning and procedure phases, in accordance with the present disclosure;
FIG. 11 is an illustration of a user interface presenting a view showing guidance of a trackable needle assembly during the procedure phase in accordance with the present disclosure; and
FIG. 12 is an illustration of a user interface presenting a view showing guidance of a surgical instrument during the procedure phase in accordance with the present disclosure.

### DETAILED DESCRIPTION

The present disclosure is directed to a system which enhances the geographic distribution of a surgical site, and assists in planning and performing a percutaneous navigation procedure. The use of a trackable needle assembly provides assistance to a clinician in optimally placing the trackable needle assembly and confirming the final placement of the trackable needle assembly. The system presents a clinician with a streamlined method of treatment planning from the initial patient selection through a process of target identification and selection, target sizing, planning for trackable needle assembly placement, route selection to create a pathway to the target, and treatment plan review. The treatment plan may then be used as a guide during placement of the trackable needle assembly during the performance of the procedure, where the system is used to track the position of the trackable needle assembly inside the patient and give the clinician a real-time view of the position of the trackable needle assembly in relation to the target and the pre-planned pathway toward the target.

In the following description, systems and methods of performing procedures will be described with reference to a percutaneous procedure; however, a person skilled in art would understand that these systems and methods could be used for performing other types of surgeries employing any percutaneous approach.

The percutaneous procedure, according to the present disclosure, is generally divided into two phases: (1) a treatment planning phase, and (2) a procedure phase. The treatment planning phase is more fully described in U.S. Patent Publication No. 2016/0038248, entitled "TREATMENT PROCEDURE PLANNING SYSTEM AND METHOD," filed on August 10, 2015 by Bharadwaj et al. The planning and procedure phase are more fully described below.

A planning and procedure system according to the present disclosure may be a unitary system configured to perform both the planning phase and the procedure phase, or the system may include separate devices and software programs for various phases. An example of the latter may be a system where a first computer device with one or more specialized software programs is used during the planning phase, and a second computing device with one or more specialized software programs may import data from the first computing device to be used during the procedure phase.

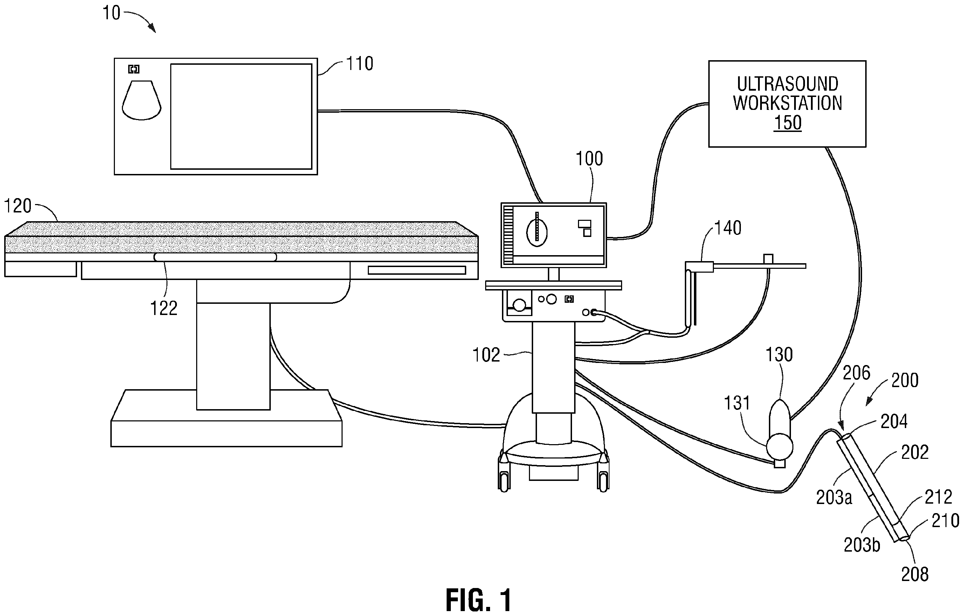

Referring now to FIG. 1, the present disclosure is generally directed to a surgical system 10, which includes a computing device 100 including a display 110 and positioned upon a microwave generator 102, a table 120, a surgical instrument 140, a trackable needle assembly 200, and an ultrasound sensor 130 connected to an ultrasound workstation 150.

Computing device 100 may be, for example, a laptop computer, desktop computer, tablet computer, or other similar device. Computing device 100 may be configured to control an electrosurgical generator, a peristaltic pump, a power supply, and/or any other accessories and peripheral devices relating to, or forming part of, system 10. Display 110 is configured to output instructions, user interfaces, images, and messages relating to the performance of the procedure. Although display 110 is shown as an integrated component of computing device 100, display 110 may be a separate component from computing device 100.

Table 120 may be, for example, an operating table or other table suitable for use during a surgical procedure. In one aspect, table 120 includes an electromagnetic (EM) field generator 122 incorporated therein. In another aspect, an EM field generator 122 is a separate component that is operably coupled to table 120. EM field generator 122 is used to generate an EM field during the percutaneous procedure and forms part of an EM tracking system which is used to track the positioning of ultrasound sensor 130, trackable needle assembly 200, and surgical instrument 140 relative to the body of a patient. EM field generator 122 may include various components, such as a specifically designed pad to be placed under, or integrated into, an operating table or patient bed. An example of such an EM tracking system is the AURORA™ EM tracking system sold by Northern Digital Inc.

Trackable needle assembly 200 includes a body portion 202, a camera 210, and a tracking sensor 212. Body portion 202 includes a proximal end 204 and a distal end 208. Body portion 202 defines lumen 206 configured to allow a surgical instrument 140 to pass therethrough. Additionally, body portion 202 may include a flexible portion 203a and a rigid portion 203b. Rigid portion 203b may be positioned distal to flexible portion 203a and be sufficiently rigid to allow percutaneous insertion of trackable needle assembly 200 into the patient. Flexible portion 203a may be sufficiently flexible to permit movement of surgical instrument 140 when it is inserted within trackable needle assembly 200. Camera 210 may be located proximate to distal end 208 of body portion 202. Camera 210 is connected to computing device 100 to display video and/or images captured by camera 210. Camera 210 may be embodied by a multiple of fiber optic cables, wherein at least one of the fiber optic cables is capable of projecting light and receiving light to capture an image. Camera 210 may be a charge coupled device (CCD) camera, a complementary metal-oxide-semiconductor (CMOS) camera, or any other appropriate camera. Sensor 212 may be located proximate to distal end 208 of body portion 202. Sensor 212 is connected to EM field generator 122 and computing device 100 to track and display the location of trackable needle assembly 200 within the patient.

Ultrasound sensor 130, such as an ultrasound wand, may be used to image the patient's body during the percutaneous procedure to visualize the locations of trackable needle assembly 200 and surgical instrument 140 inside the patient's body. Ultrasound sensor 130 includes an EM tracking element 131 embedded within or attached to ultrasound sensor 130, for example, a clip-on sensor or a sticker sensor. As described further below, ultrasound sensor 130 may be positioned in relation to trackable needle assembly 200 such that trackable needle assembly 200 is at an angle relative to the ultrasound image plane, thereby enabling the clinician to visualize the spatial relationship of trackable needle assembly 200 with the ultrasound image plane and with objects being imaged. Ultrasound workstation 150 may be used to configure, operate, and view images captured by ultrasound sensor 130.

Surgical system 10 additionally includes surgical instrument 140 which is also trackable by computing device 100 via EM field generator 122. To this end surgical instrument 140 includes a trackable element dissimilar to that of which is used with ultrasound sensor 130 and trackable needle assembly 200. Surgical instrument 140 is positionable through trackable needle assembly 200 to gain access to the surgical site. Surgical instrument 140 may be any type of therapeutic, treatment, or surgical device, including for example, an ablation device, a biopsy device, a marker placement device, or any other such device.

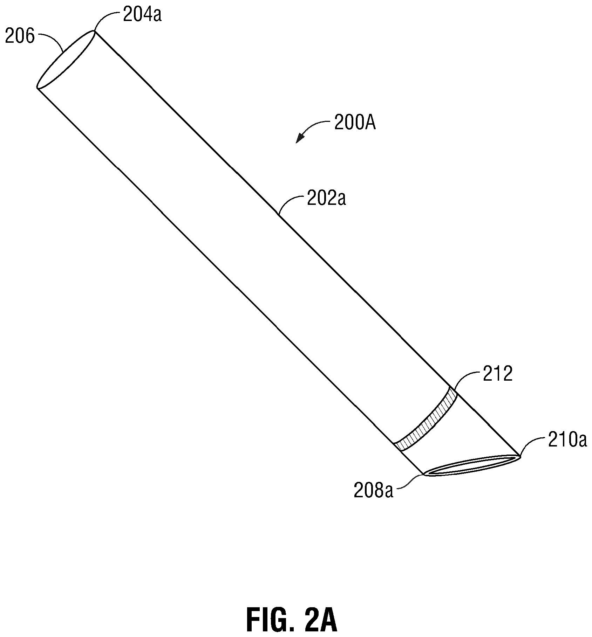

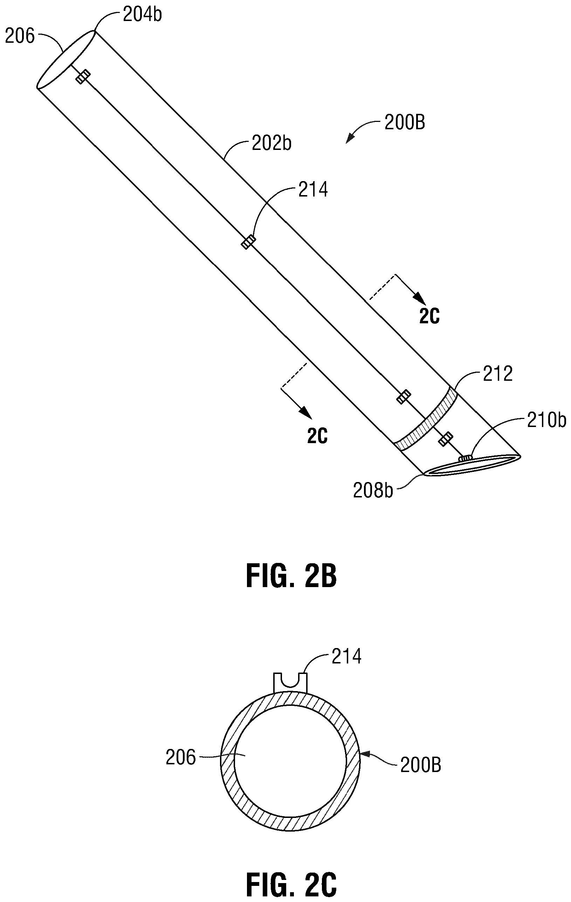

Turning now to FIGS. 2A-2C, embodiments according to the present invention 2. of trackable needle assembly 200 are illustrated and described as trackable needle assembly 200A (FIG. 2A) and trackable needle assembly 200B (FIG. 2B).

Trackable needle assembly 200A includes a camera 210a integrally formed therewith. Camera 210a may be located proximate to distal end 208a of body 202a.

Trackable needle assembly 200B includes a camera 210b and camera guiding features 214. Camera 210b is attached to body 202b via camera guiding features 214 such that camera 210b is proximate to distal end 208b of body 202b. Camera guiding features 214 are positioned along body 202b of trackable needle assembly 200B. Camera guiding features 214 may be a U-shaped snap feature that is configured to secure camera 210b to body 202b of trackable needle assembly 200B (FIG. 2C). Camera guiding features 214 may be evenly spaced apart from one another and/or may be unevenly spaced apart from one another.

The location of trackable needle assembly 200 within the body of the patient may be tracked during the surgical procedure. Although illustrated and described as being used with one trackable needle assembly (and one surgical instrument), it is understood that multiple trackable needle assemblies may be used with surgical system 10 and multiple trackable needle assemblies (and surgical instruments) may be displayed. An example method of tracking the location of trackable needle assembly 200 is by using EM tracking system 122 (FIG. 1), which tracks the location of trackable needle assembly 200 by tracking sensors 212 attached to or incorporated in trackable needle assembly 200. Various types of sensors may be used, such as a printed sensor, the construction and use of which is more fully described in co-pending U.S. Patent Publication No. 2016/0174873, entitled "MEDICAL INSTRUMENT WITH SENSOR FOR USE IN A SYSTEM AND METHOD FOR ELECTROMAGNETIC NAVIGATION," filed October 22, 2015 by Greenburg et al.

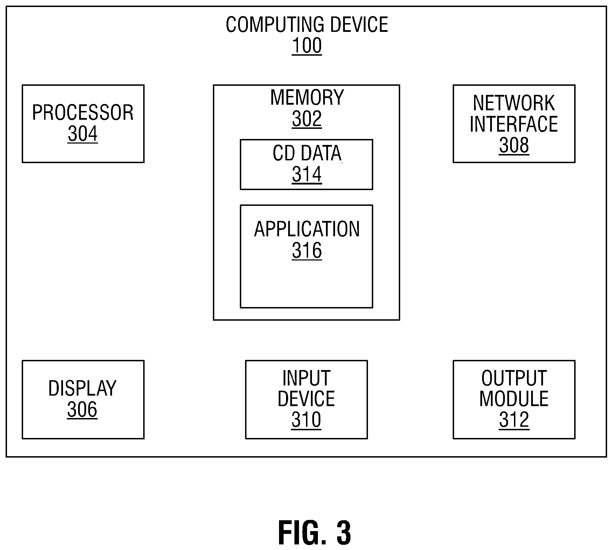

FIG. 3 depicts a system diagram of computing device 100 of surgical system 10 (FIG. 1). Computing device 100 may include memory 302, processor 304, display 306 (or display 110), network interface 308, input device 310, and/or output module 312.

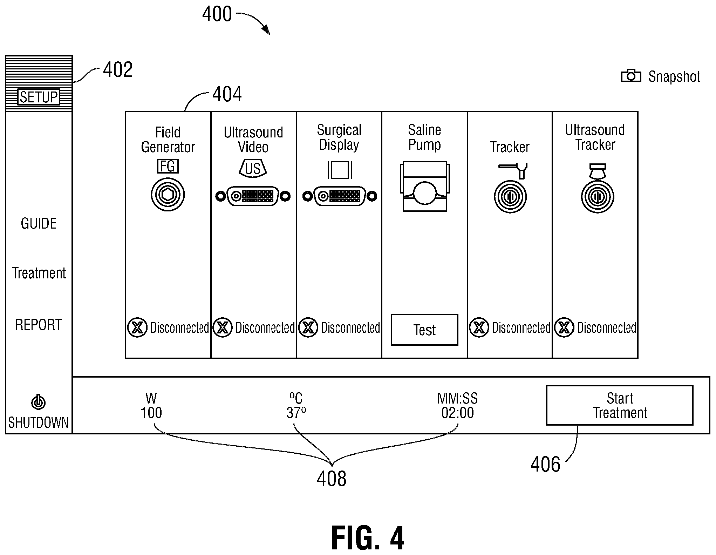

Memory 302 includes any non-transitory computer-readable storage media for storing data and/or software (e.g., application 316) that is executable by processor 304 and which controls the operation of computing device 100. In an embodiment, memory 302 may include one or more solid-state storage devices such as flash memory chips. Memory 302 may store application 316 and/or CT data 314. Application 316 may, when executed by processor 304, cause display 306 and/or display 110 (FIG. 1) to present user interfaces, such as the user interfaces illustrated in FIGS. 4, 5, 7-9, 11, and 12.

Processor 304 may be a general purpose processor, a specialized graphics processing unit (GPU) configured to perform specific graphics processing tasks while freeing up the general purpose processor to perform other tasks, and/or any number or combination of such processors.

Display 306 may be touch sensitive and/or voice activated, enabling display 306 to serve as both an input and output device. Alternatively, a keyboard (not shown), mouse (not shown), or other data input devices may be employed.

Network interface 308 may be configured to connect to a network such as a local area network (LAN) consisting of a wired network and/or a wireless network, a wide area network (WAN), a wireless mobile network, a Bluetooth network, and/or the internet. For example, computing device 100 may receive computed tomographic (CT) image data 314 of a patient from a server, for example, a hospital server, internet server, or other similar servers, for use during planning of the procedure phase. Patient CT image data 314 may also be provided to computing device 100 via a removable memory (not illustrated). Computing device 100 may receive updates to its software, for example, application 316, via network interface 308. Computing device 100 may also display notifications on display 306 that a software update is available.

Input device 310 may be any device by means of which a user may interact with computing device 100, such as, for example, a mouse, keyboard, foot pedal, touch screen, and/or voice interface.

Output module 312 may include any connectivity port or bus, such as, for example, parallel ports, serial ports, universal serial busses (USB), or any other similar connectivity port known to those skilled in the art.

Application 316 may be one or more software programs stored in memory 302 and executable by processor 304 of computing device 100. As will be described in more detail below, during the planning phase, application 316 guides the clinician through a series of steps to identify a target, the size of the target, and/or determine an access route to the target for later use during the procedure phase.

In some embodiments, application 316 is loaded on computing devices in an operating room or other facility where surgical procedures are performed, and is used as a plan or map to guide a clinician performing a surgical procedure, but without any feedback from trackable needle assembly 200 used in the procedure to indicate where trackable needle assembly 200 is located in relation to the plan. In other embodiments, system 10 provides computing device 100 with data regarding the location of trackable needle assembly 200 within the body of the patient, such as by EM tracking, which application 316 may then use to indicate on the plan where trackable needle assembly 200 is located. Application 316 may be installed directly on computing device 100, or may be installed on another computer, for example a central server, and opened and operated on computing device 100 via network interface 308.

Having described the components of surgical system 10 depicted in FIGS. 1-3, the following description of FIGS. 4-12 provide exemplary workflows of using the components of surgical system 10 and user interfaces thereof. The systems and methods described herein may be useful for visualizing a particular target region of a patient and navigating electromagnetically trackable needle assemblies thereto. Although the methods illustrated and described herein as being in a particular order and requiring particular steps, any of the methods may include some or all of the steps and may be implemented in any order not specifically described. Additionally, although the methods are described as being carried out by computing device 100, any component of computing device 100 or surgical system 10 may carry out any or all of the steps described in the methods below.

Turning now to FIG. 4, an exemplary user interface which may be displayed on display 306 and/or display 110 is illustrated and referred to herein as user interface 400. User interface 400 shows an indicator 402 representing the progress of the percutaneous procedure. User interface 400 also includes a list 404 which indicates various system components which should be connected for the procedure, as well as the status of those components. A button 406 is provided when a system component is connected to test the functioning of that component. User interface 400 also shows indicators 408 representing the configured parameters of the system, trackable needle assembly 200, and surgical instrument 140.

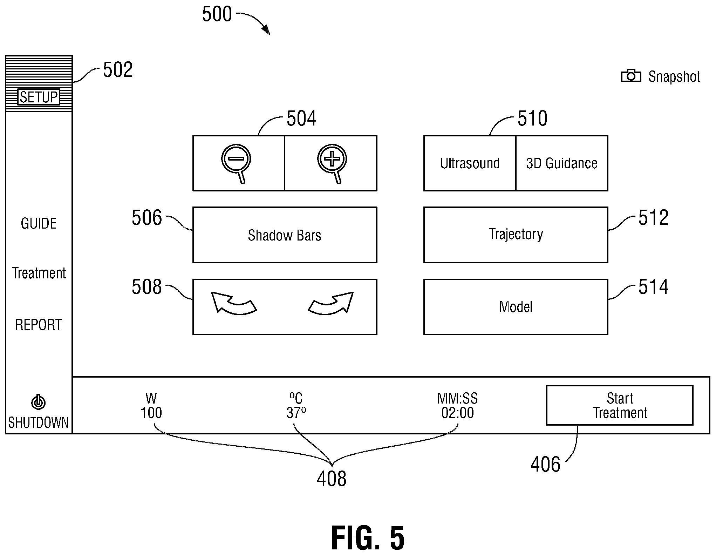

Referring now to FIG. 5, an example user interface 500 which may be displayed on display 306 either during the guidance step of trackable needle assembly 200 and/or selected surgical instrument 140 or selected at any time by the clinician to adjust the features of the system 10. User interface 500 shows an indicator 502 that the system is now operating in the guidance step. User interface 500 further provides buttons 504 allowing the clinician to zoom in and out on the model and pathway displayed on display 110. User interface 500 further provides a button 506 which enables a shadow bar overlay on the pathway displayed on display 110 which indicates whether the trajectory of trackable needle assembly 200 and/or selected surgical instrument 140 is in front of or behind an ultrasound image plane within the guidance view displayed on display 110. This enables the clinician to visualize the projected trajectory of trackable needle assembly 200 and/or selected surgical instrument 140, as well as the interaction of the trajectory of trackable needle assembly 200 and/or selected surgical instrument 140 within, or related to, the ultrasound image plane.

User interface 500 also includes buttons 508 allowing the clinician to rotate the guidance view displayed on display 110. User interface 500 further includes a button 510 allowing the clinician to toggle between a view of the model with the pathway and a live ultrasound image video feed. User interface 500 also includes a button 512 allowing the clinician to toggle the display of the planned pathway of trackable needle assembly 200 and/or selected surgical instrument 140 on the model, and a button 514 allowing the clinician to toggle the display of a projected treatment zone relative to trackable needle assembly 200 and/or selected surgical instrument 140 on the model to enable the clinician to visualize the treatment zone relative to trackable needle assembly 200 and/or selected surgical instrument 140. The treatment zone may also be overlaid on the ultrasound images, thereby allowing the clinician to visualize the treatment zone within the ultrasound plane. The treatment zone may be presented to the clinician in a 2D and 3D treatment zone model.

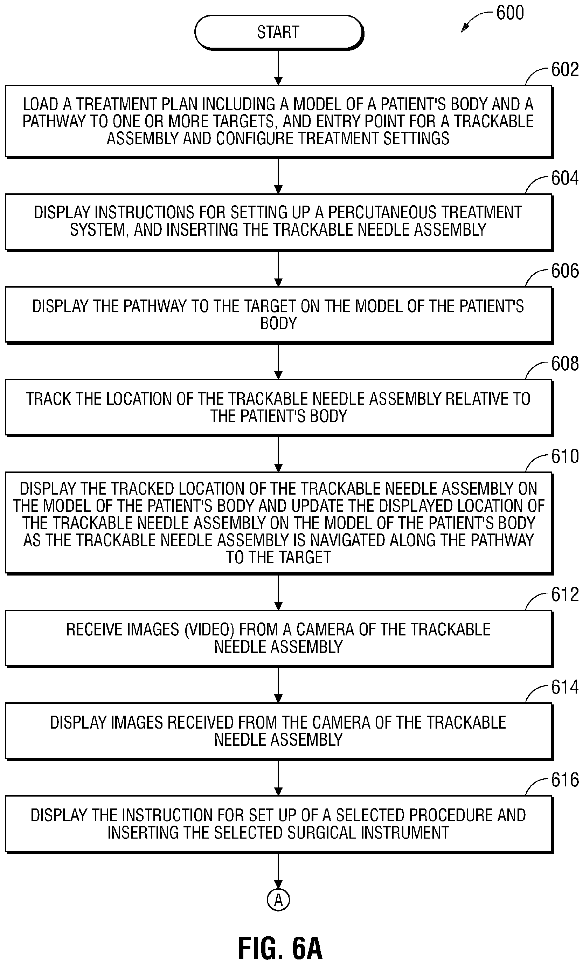

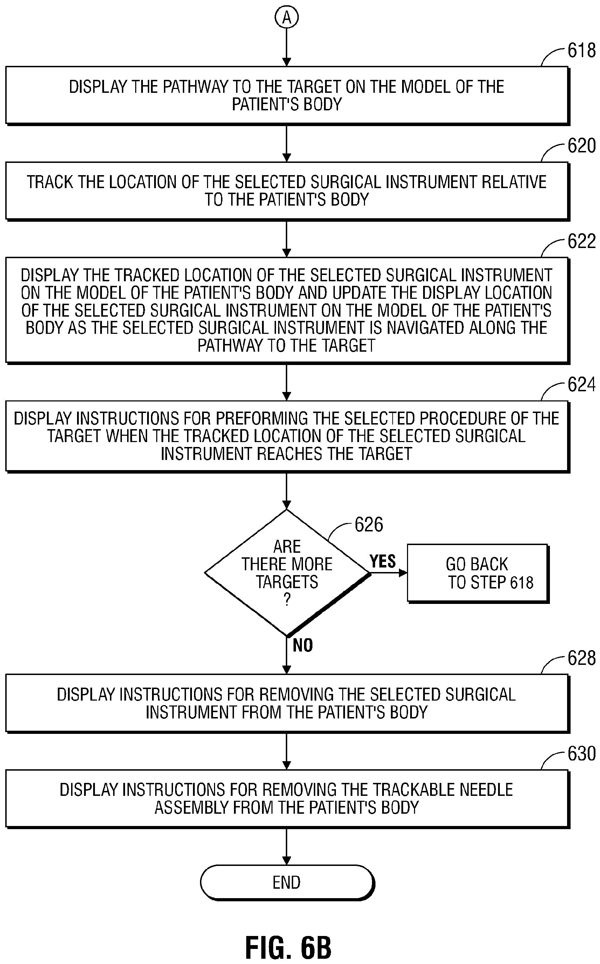

Turning now to FIG. 6, a method for performing a percutaneous procedure using trackable needle assembly 200 and a patient model is illustrated and will be referred to as method 600. Method 600 begins at step 602 where treatment plan is loaded into computing device 100. The treatment plan loaded in step 602 includes a model of a patient's body (e.g. a three-dimensional model), and may additionally include a pathway to one or more targets, possible procedures, and possible surgical instruments usable in the procedures. Further, the treatment plan loaded in step 602 includes an entry point for trackable needle assembly 200 to one or more targets depending on the possible procedures, and possible surgical instruments usable in the procedures.

The model and treatment plan loaded into computing device 100 in step 602 are both generated during the planning phase. The model may be generated based on CT image data acquired during a CT scan of the patient, although other imaging modalities are also envisioned. The clinician uses the model to select one or more targets for treatment during the percutaneous procedure. The clinician also uses the model to select the procedure and surgical instruments that will be used for treatment during the percutaneous procedure. Thereafter, computing device 100 generates a pathway from each selected target to entry point(s) on the patient's body where a trackable needle assembly 200 may be inserted. The pathway and point of entry are also generated in such a way as to avoid any bones, vital organs, or other critical structures inside the patient's body. After loading the treatment plan on computing device 100, the clinician may view and modify the treatment plan. The clinician may further configure the system settings for the procedure. For example, the clinician may preconfigure parameters related to the various tools to be used during the procedure.

At step 604, instructions for setting up and configuring the percutaneous treatment system are displayed on user interface 400 (FIG. 4) of display 306. The instructions may be visual and/or audible, and may provide feedback for proper versus improper system configuration. When the system has been configured for the procedure, the clinician may start the procedure, stop the procedure, pause the procedure, resume the procedure, and/or reset the procedure by selecting a button 406 of user interface 400 (FIG. 4). Upon selecting button 406, computing device 100 starts one or more of the system components and/or operations. For example, application 316 may automatically start a peristaltic pump, an electrosurgical generator, and/or a power supply. Then, instructions for inserting trackable needle assembly 200 into the patient's body are displayed on display 306. Thereafter, at step 606, the model of the patient's body with the pathway to target as was generated in the planning phase is displayed on display 306.

In one embodiment, the treatment phase is similar to that employed by the iLogic® system currently sold by Medtronic, in which the position of the patient in the magnetic field is registered with the images from the planning phase. In addition, the location of trackable needle assembly 200 in the electromagnetic field is detected and displayed with reference to the planned pathway and the position of the patient and more specifically with respect to the target identified and displayed in the model. In another embodiment, the display may display real time video being captured by camera 210 of trackable needle assembly 200. The real time video and tracking of trackable needle assembly 200 in the electromagnetic field may be simultaneously displayed and considered by the clinician.

In step 608, while trackable needle assembly 200 is navigated by the clinician, the location of trackable needle assembly 200 relative to the patient's body is tracked by computing device 100. In particular, computing device 100 utilizes the positional data generated by electromagnetic transmitter 122 (FIG. 1) and sensor 212 of trackable needle assembly 200 to determine the relative position of trackable needle assembly 200.

In step 610, the location of trackable needle assembly 200 (tracked in step 608) is displayed on the model of the patient's body which was loaded into computing device 100 in step 602. In addition, a vector is projected extending from the end of trackable needle assembly 200 to give an indication to the clinician of the intersecting tissue along the trajectory of trackable needle assembly 200. In this manner, the clinician can alter the approach of inserting trackable needle assembly 200 to optimize placement with a minimum amount of trauma. Display 110 and/or display 306 may be a split screen display, where the tracked location of trackable needle assembly 200 on the model of the patient's body (generated in step 608) is displayed in one portion of the user interface and real time video being captured by camera 210 of trackable needle assembly 200 is displayed on another portion of the same user interface.

Further in step 610, computing device 100 iteratively updates the displayed location of trackable needle assembly 200 on the model of the patient's body as trackable needle assembly 200 is navigated along the pathway to the target.

When trackable needle assembly 200 has reached the desired proximity to the target, computing device 100 may automatically detect when a portion of trackable needle assembly 200 is within a given distance from the target and may notify the clinician of such a detection.

At step 612, video and/or images are received from camera 210 of trackable needle assembly 200. Camera 210 may begin capturing videos and/or images prior to insertion of trackable needle assembly 200 and can continue to capture videos and/or images while trackable needle assembly 200 is moved proximate to the targeted area. Thereafter, at step 614, computing device 100 displays the videos and/or images received. These videos and/or images may be viewed simultaneously to the model of the patient's body generated in step 608.

At step 616, computing device 100 displays instructions for the selected procedure, including a list of steps for the selected procedure and a list of the selected instruments that are required to perform each step of the selected procedure. Thereafter, at step 618, the model of the patient's body with the pathway to target as was generated in the planning phase is again displayed.

In step 620, while the selected surgical instrument 140 is navigated, the location of the surgical instrument 140 is tracked. In step 622, the tracked location of selected surgical instrument 140 (from step 620) is displayed on the model of the patient's body which was loaded in step 602. In addition, a vector is projected extending from the end of selected surgical instrument 140 to give an indication to the clinician of the intersecting tissue along the trajectory of selected surgical instrument 140. In this manner, the clinician can alter the approach of transitioning surgical instrument 140 to the target to minimize trauma in cases where distal end 208 of trackable needle assembly 200 does not reach the target.

Further, at step 622, the location of selected surgical instrument 140 is iteratively updated and displayed on the model of the patient's body as selected surgical instrument 140 is navigated along the pathway to the target.

At step 624, when the clinician detects that selected surgical instrument 140 has reached the target, the instructions for the selected procedure, including the parameters of selected surgical instrument 140 previously set by the clinician for treating the target are displayed, and the clinician may select the "start treatment" button to treat the target. For example, when the clinician selects the "start treatment" surgical instrument 140 may ablate, extract a sample, or perform any other appropriate treatment to the target. When the "start treatment" button is selected, system 10 may automatically start other related accessories and/or peripheral devices, such as an associated peristaltic pump. The videos and/or images of camera 210 and the tracked location of trackable needle assembly 200, surgical instrument 140, and the model of the patient's body generated in step 608 may be continuously updated and simultaneously viewed throughout the entire duration of the treatment of the target.

Thereafter, at step 626 it is determined if there are any more targets in the treatment plan that have yet to be treated based on the planned procedure. If the determination is yes, the process returns to step 618 where the displayed pathway is updated to reflect the pathway to the next target. If the determination is no, at step 628, instructions are displayed for removing selected surgical instrument 140 from the patient's body. At step 630, instructions are displayed for removing trackable needle assembly 200 from the patient's body. During the selected procedure, data relating to parameters of trackable needle assembly 200, selected surgical instrument 140 and selected procedure, such as degree of insertion, distance from the target, optimal triangulation, power, time settings, and temperature, is continually stored. Additionally, application 316 may present the clinician with instructions, such as a workflow, relating to protocols associated with the selected procedure.

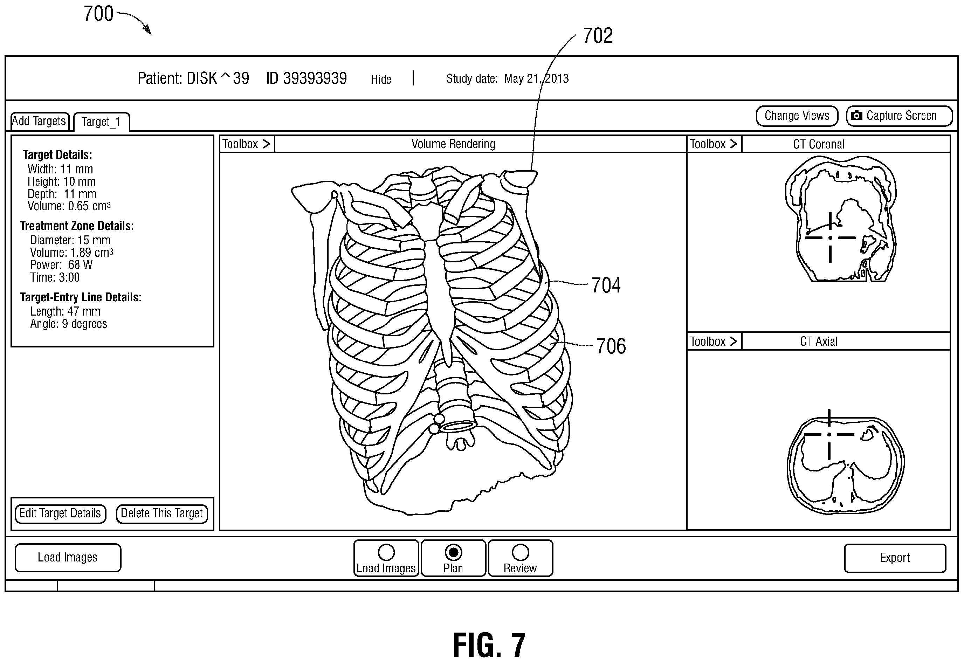

FIGS. 7, 8, and 10 show examples of a user interface 700 which may be displayed on display 110 during the percutaneous procedure. The 3D model 702 provides the clinician with a representation of the patient's anatomy and, in an exemplary embodiment, a representation of the patient's chest and thoracic cavity, as shown in FIG. 7. The 3D model 702 presents the clinician with multiple layers of the patient's anatomy including, for example, representations of the patient's skin, muscle, blood vessels, bones, airways, lungs, other internal organs, or other features of the patient's anatomy. For example, as shown in FIG. 7, a 3D model 702 of the patient's thoracic cavity with the outer layer peeled back, removed, or adjusted to present a layer including the patient's ribs 704 and layers including other anatomical features 706 of the patient's internal anatomy to the clinician. Layers 704, 706 may be presented at different levels of opacity or transparency to allow the clinician to review the interior of the patient's torso relative to the target area. 3D model 702 may be rotated by activating a user input to allow the clinician to view the treatment plan at various angles and directions. The clinician may also activate a user input to peel back, remove, or adjust the opacity and translucence of each layer of the 3D model to provide the clinician with a visual representation of the planned entry route to the target area relative to surrounding critical structures within the patient's body.

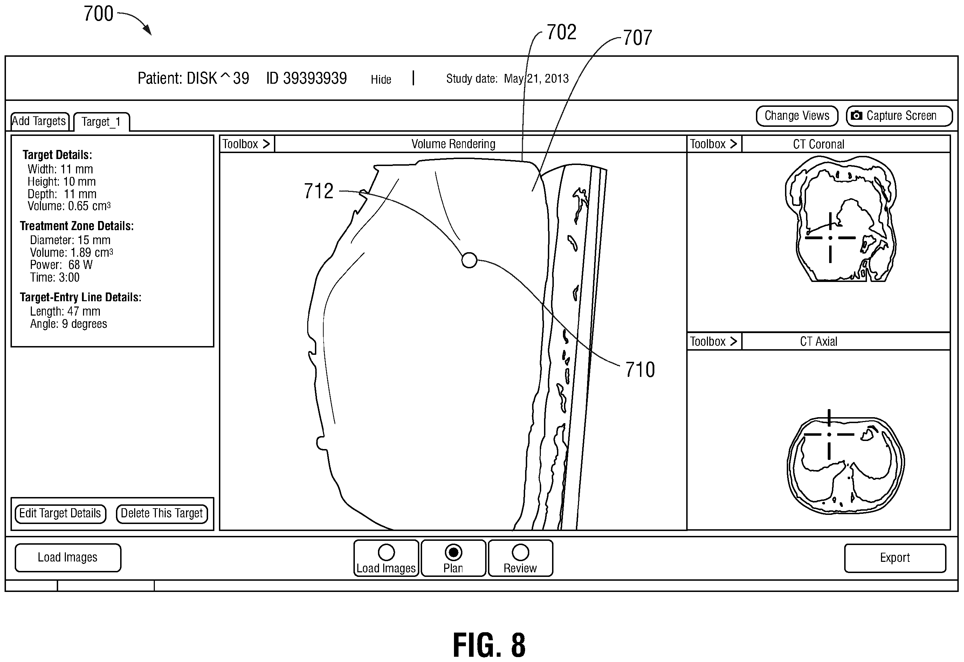

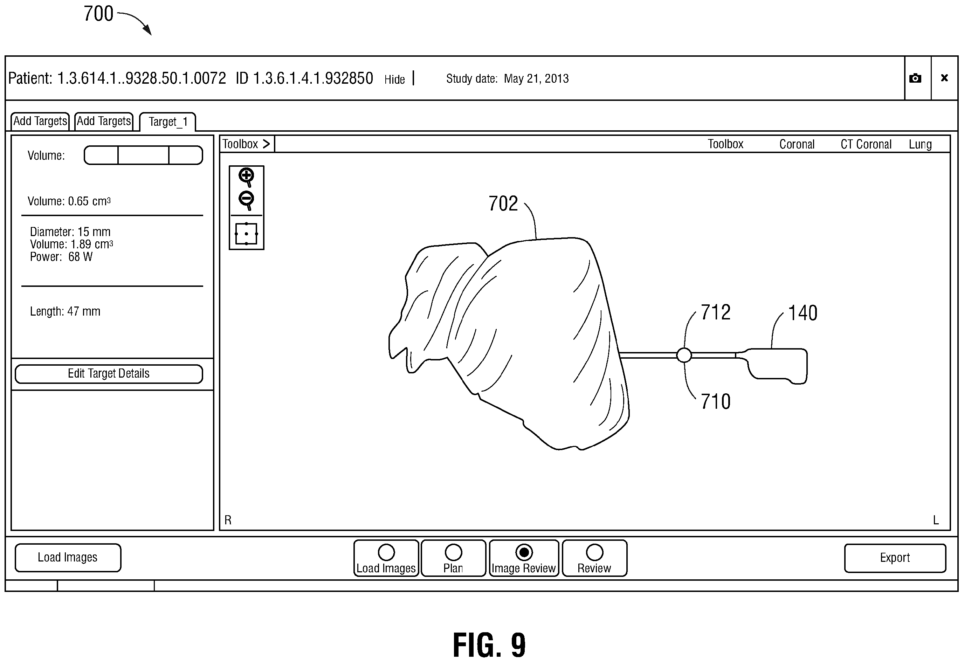

As seen in FIG. 8, the patient's chest is presented with 3D model 702 including a representation of the patient's skin 707 overlaid over the patient's rib cage 704 (FIG. 7) and other anatomical features 706 (FIG. 7) such that an end point 712 and the entry route marker 710 are shown exiting the representation of the patient's body. The end point 712 and the entry route marker 710 may also be presented as a representation of trackable needle assembly 200, selected surgical instrument 140, as shown in FIG. 9.

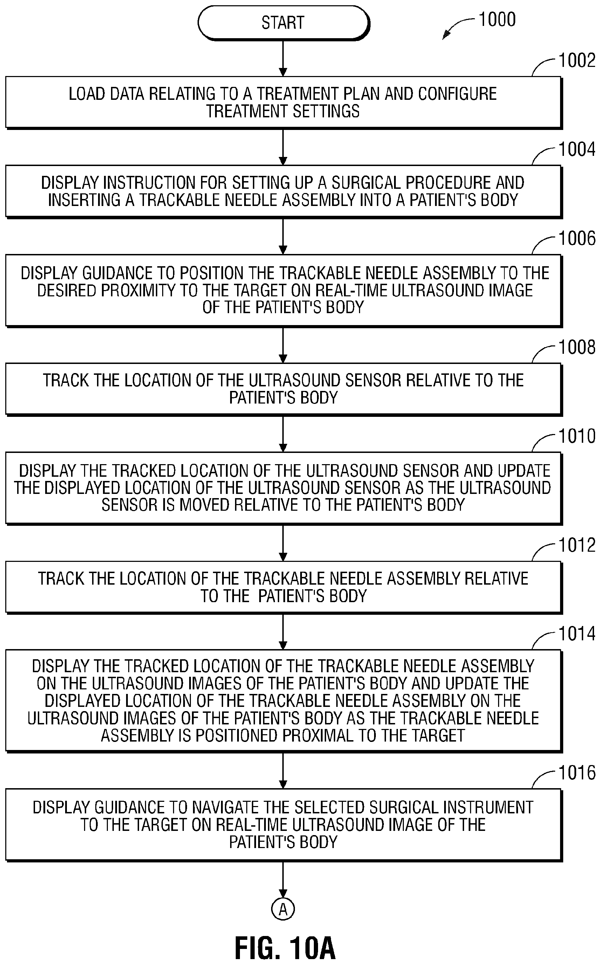

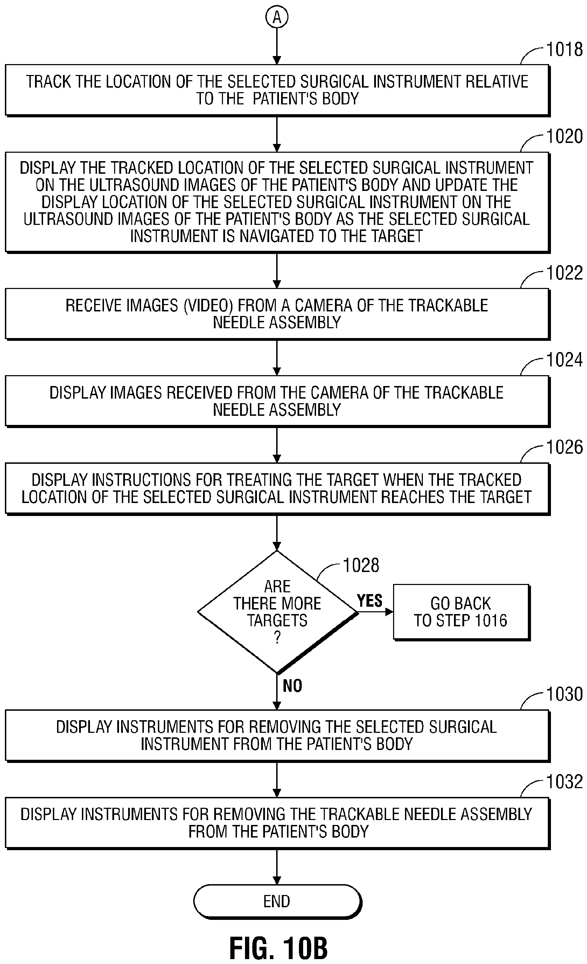

In some embodiments, system 10 may be operated without using the model generated during the planning phase of the percutaneous procedure. In such embodiments, placement of trackable needle assembly 200 and navigation of selected surgical instrument 140 are guided by using ultrasound images, such as the ultrasound images generated by ultrasound sensor 130. During the guidance step of the percutaneous procedure, the location of trackable needle assembly 200, selected surgical instrument 140 and the one or more targets are overlaid onto the ultrasound images generated by ultrasound sensor 130. By doing so, the location of trackable needle assembly 200 and selected surgical instrument 140 may be viewed in relation to the ultrasound image plane to visualize a trajectory of trackable needle assembly 200 and selected surgical instrument 140. The location of trackable needle assembly 200 and selected surgical instrument 140 may be tracked by the EM tracking system 122, while the location of the one or more targets are determined based on data generated during the planning phase. A vector may also be displayed from the tip of trackable needle assembly 200, showing the trajectory of trackable needle assembly 200 and allowing the clinician to align trackable needle assembly 200 to the target. Additionally, a vector may also be displayed from the tip of the selected surgical instrument 140, showing the trajectory of the selected surgical instrument 140 and allowing the clinician to align selected surgical instrument 140 to the target. An example method of performing a percutaneous procedure according to this embodiment is described below with reference to FIG. 10.

Referring now to FIG. 10, a flowchart of an example method for performing a percutaneous procedure according to an embodiment of the present disclosure is illustrated and will be referred to as method 1000. Method 1000 begins at step 1002 where the clinician may use computing device 100 to load data relating to a treatment plan into application 316. The data may include the location of one or more targets within a patient's body, and a pathway to the one or more targets.

At step 1004, instructions for setting up and configuring the percutaneous procedure, and inserting trackable needle assembly 200 into the patient's body, are displayed on user interface 400 (FIG. 4) of display 306. Additionally, a list of appropriate procedures that may be performed to treat the patient, and a list of appropriate surgical instrument that the clinician can use in performing the selected procedure are displayed on user interface 400 of display 306. Both the procedure and surgical instruments may be selected during the planning phase and loaded with the other data relating to the treatment plan into application 316. Alternatively, both the procedure and surgical instruments may be selected in step 1004. While configuring the system, the clinician may select the procedure and surgical instrument via the user interface 400.

In step 1006, computing device 100 displays guidance to position trackable needle assembly 200 to the desired proximity to the target on ultrasound images generated by ultrasound sensor 130 on user interface 400 of display 306. The displayed guidance may include instructions for insertion of more than one trackable needle assembly 200 to access one or more targets and/or a graphical map or pathway to the one or more targets which may be overlaid onto the ultrasound images.

In step 1008, the location of ultrasound sensor 130 is tracked in relation to the patient's body by computing device 110. In particular, computing device 100 utilizes the positional data generated by electromagnetic transmitter 122 (FIG. 1) and tracking element 131 of ultrasound sensor 130 to determine the relative position of ultrasound sensor 130.

In step 1010, the location and relative position of ultrasound sensor 130 (tracked in step 1008) are displayed on user interface 1100. The display of user interface 1100 displays the updated location and relative position of ultrasound sensor 130 as ultrasound sensor 130 is moved relative to the patient's body.

In step 1012, trackable needle assembly 200 is navigated by the clinician to the desired proximity to the target and the location of trackable needle assembly 200 inside the patient's body is tracked. At step 1014, computing device 100 displays the tracked location of trackable needle assembly 200 on the ultrasound images of the patient's body generated by ultrasound sensor 130. Computing device 100 displays and iteratively updates of the location of trackable needle assembly 200 on the ultrasound images as trackable needle assembly 200 is navigated to the target.

In step 1016, guidance to navigate surgical instrument 140 to the target on ultrasound images generated by ultrasound sensor 130 is displayed. The displayed guidance may include instructions for navigating the surgical instrument 140 to the one or more targets and/or a graphical map or pathway to the one or more targets which may be overlaid onto the ultrasound images.

In step 1018, the clinician navigates the selected surgical instrument 140 to the target. While selected surgical instrument 140 is navigated, the location of surgical instrument 140 inside the patient's body is tracked. In step 1020, computing device 100 displays the tracked location of surgical instrument 140 on the ultrasound images of the patient's body generated by ultrasound sensor 130. Computing device 100 displays and iteratively updates the location of surgical instrument 140 on the ultrasound images as surgical instrument 140 is navigated to the target.

At step 1022, video and/or images are received from camera 210 of trackable needle assembly 200. Camera 210 may begin capturing videos and/or images prior to insertion of trackable needle assembly 200 and can continue to capture videos and/or images while trackable needle assembly 200 is moved proximate to the targeted area. The videos and/or images of camera 210 may assist the clinician in evaluating the progression of the treatment by providing visualization of the target area. Thereafter, at step 1024, computing device 100 displays the videos and/or images received. These videos and/or images may be viewed simultaneously to the ultrasound images generated by ultrasound sensor 130.

At step 1026, computing device 100 displays instruction for treating the target when the tracked location of selected surgical instrument 140 reaches the target. Thereafter, at step 1028, it is determined if there are any more targets in the treatment plan that have yet to be treated based on the planned procedure. If the determination in step 1028 is yes, the process returns to step 1016 where the displayed pathway is updated to reflect the pathway to the next target. If the determination in step 1028 is no, then computing device 100 displays instructions for removing selected surgical instrument 140 from the patient's body (step 1030). At step 1032, the application displays instructions for removing trackable needle assembly 200 from the patient's body. During the selected procedure, data relating to parameters of trackable needle assembly 200, selected surgical instrument 140 and selected procedure, such as degree of insertion, distance from the target, optimal triangulation, power, time settings, and temperature, is continually stored. Additionally, the clinician may be presented with instructions, such as a workflow, relating to protocols associated with the selected procedure.

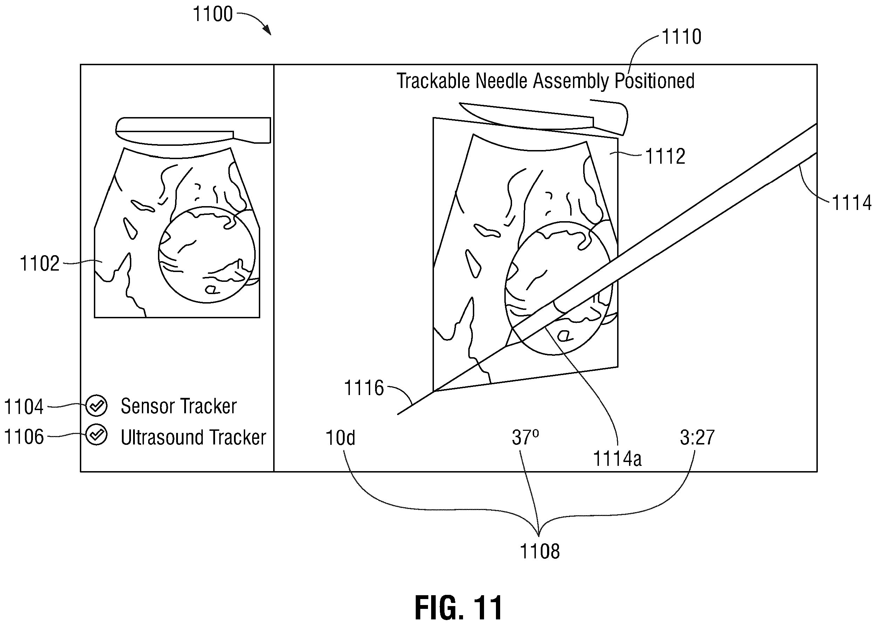

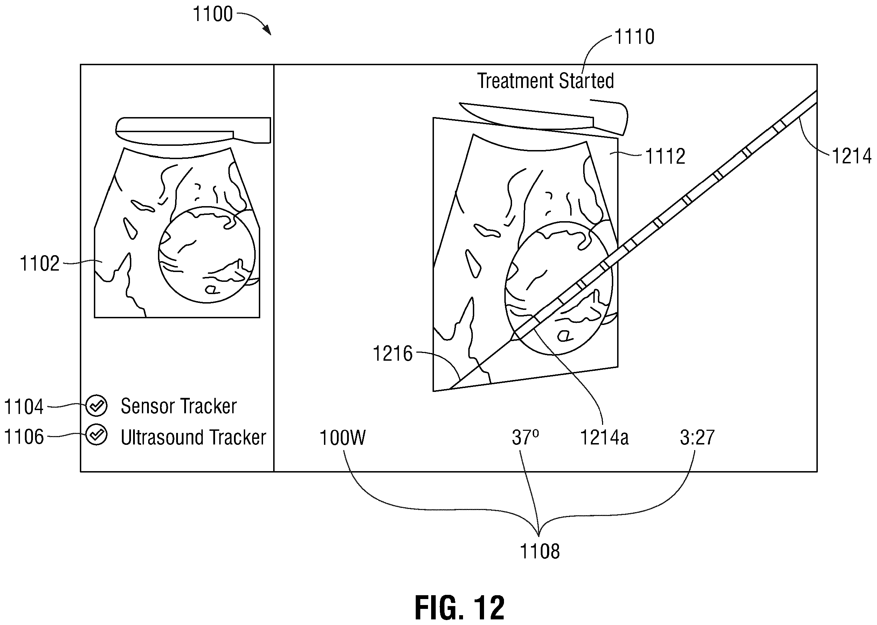

FIGS. 11 and 12 show examples user interface 1100 which may be displayed on display 110 during the procedure. User interface 1100 includes a view 1102 of the live 2D ultrasound images captured during the procedure. User interface 1100 further shows a status indicator 1104 for trackable needle assembly 200 and selected surgical instrument 140 and a status indicator 1104 for ultrasound sensor 130. User interface 1100 also includes a view 1108 for displaying status messages relating to the percutaneous procedure, such as the angle of insertion of trackable needle assembly 200, the degree of misalignment of trackable needle assembly 200 from the planned pathway, the depth of trackable needle assembly 200, parameter of selected surgical instrument 140, duration of the selected procedure and/or a time remaining until the selected procedure is complete, progression of the selected procedure, feedback from a temperature sensor, and a treatment zone chart used during the selected procedure (FIG. 11). User interface 1100 further includes a view 1110 for showing transient messages relating to the percutaneous procedure, such as changes caused by selecting the buttons provided by user interface 400, described above. User interface 1100 also displays the navigation view 1112, which includes a representation 1114 of trackable needle assembly 200 (FIG. 11) and a representation 1214 of selected surgical instrument 140 (FIG. 12) as well as a shadow indicator 1114a representing the portion of trackable needle assembly 200 (FIG. 11) and a shadow indicator 1214a representing the portion of selected surgical instrument 140 (FIG. 12) which lies below the ultrasound imaging plane, a vector line 1116, 1216 representing the trajectory of trackable needle assembly 200 and selected surgical instrument 140, respectively.

Further to the above-description, the creation of a virtual and real time volume visible to a clinician/user is displayed by system 10 whereby the display can be based on pre-procedural and/or intra-procedural imaging. The imaging data (even static data) can be displayed three dimensionally where several trackable tools (or trackable needle assemblies) are then superimposed on those images in real time. This would further allow for the clinician/user to assess for things like proximity to other critical structures or whereby either a clinical assessment can be made leading to the recognition of the need for certain tools or whereby suggestions are made to the clinician by the system 10 given the geometry of different tools for the various positions and angles that might be required to effect the procedure or the treatment.

It will be apparent to those of ordinary skill in the art that various modifications to the foregoing embodiments may be made without departing from the scope of the accompanying claims.

## Claims

1. A system (10) for performing a percutaneous navigation procedure, the system comprising:
a trackable needle assembly (200) including a first tracking sensor (212) disposed thereon and defining a lumen (206) therethrough;
a camera (210) attached to the trackable needle assembly, the camera configured to capture video or images;
a surgical instrument (140) including a second tracking sensor disposed thereon, the surgical instrument configured to be inserted through the lumen (206) of the trackable needle assembly (200);
an ultrasound sensor (130) including a tracking element (131) disposed thereon; and
a computing device (100) configured to:
track a location of each of the ultrasound sensor (130), the trackable needle assembly (200), and the surgical instrument (140); and
display the location of each of the ultrasound sensor, the trackable needle assembly, and the surgical instrument in relation to one another.

2. The system for performing a percutaneous navigation procedure of claim 1, wherein the computing device (100) is configured to:
receive video or images captured by the camera (210); and
display the received video or images.

3. The system for performing a percutaneous navigation procedure of claim 2, wherein the displayed video or images of the camera (210) is configured to be continuously updated as the trackable needle assembly (200) is navigated.

4. The system for performing a percutaneous navigation procedure of any preceding claim, further comprising an electromagnetic field generator (122) configured to generate an electromagnetic field to be sensed by the first tracking sensor (212) of the trackable needle assembly (200), the second tracking sensor of the surgical instrument (140), and the tracking element (131) of the ultrasound sensor (130).

5. The system for performing a percutaneous navigation procedure of any preceding claim, wherein the computing device (100) is configured to update the displayed location of the ultrasound sensor (130), the trackable needle assembly (200), and the surgical instrument (140) in relation to one another as each of the ultrasound sensor, the trackable needle assembly, and the surgical instrument are moved.

6. The system for performing a percutaneous navigation procedure of any preceding claim, wherein the camera (210) of the trackable needle assembly (200) is integrally formed with the trackable needle assembly.

7. The system for performing a percutaneous navigation procedure of any of claims 1 to 5, wherein the camera (210) of the trackable needle assembly (200) is detachable connected to the trackable needle assembly.

8. The system for performing a percutaneous navigation procedure of claim 7, wherein the trackable needle assembly (200) includes a plurality of camera guiding features (214) configured to connect the camera (210) to the trackable needle assembly.

## Patentansprüche

1. System (10) zum Durchführen eines perkutanen Navigationsverfahrens, wobei das System Folgendes umfasst:
eine verfolgbare Nadelanordnung (200) einschließlich eines ersten Verfolgungssensors (212), der darauf angeordnet ist und ein Lumen (206) dahindurch definiert;
eine Kamera (210), die an der verfolgbaren Nadelanordnung angebracht ist, wobei die Kamera konfiguriert ist, um Videos oder Bilder aufzunehmen;
ein chirurgisches Instrument (140) einschließlich eines zweiten Verfolgungssensors, der darauf angeordnet ist, wobei das chirurgische Instrument konfiguriert ist, um durch das Lumen (206) der verfolgbaren Nadelanordnung (200) eingeführt zu werden;
einen Ultraschallsensor (130) einschließlich eines Verfolgungselements (131), der darauf angeordnet ist; und
eine Rechenvorrichtung (100), die für Folgendes konfiguriert:
Verfolgen einer Lage jeweils des Ultraschallsensors (130), der verfolgbaren Nadelanordnung (200) und des chirurgischen Instruments (140); und
Anzeigen der Lage jeweils des Ultraschallsensors, der verfolgbaren Nadelanordnung und des chirurgischen Instruments in Bezug zueinander.

2. System zum Durchführen eines perkutanen Navigationsverfahrens nach Anspruch 1, wobei die Rechenvorrichtung (100) für Folgendes konfiguriert ist:
Empfangen von Videos oder Bildern, die durch die Kamera (210) aufgenommen werden; und
Anzeigen der empfangenen Videos oder Bilder.

3. System zum Durchführen eines perkutanen Navigationsverfahrens nach Anspruch 2, wobei das angezeigte Video oder die angezeigten Bilder der Kamera (210) konfiguriert sind, um fortlaufend aktualisiert zu werden, während die verfolgbare Nadelanordnung (200) navigiert wird.

4. System zum Durchführen eines perkutanen Navigationsverfahrens nach einem der vorhergehenden Ansprüche, ferner umfassend einen elektromagnetischen Feldgenerator (122), der konfiguriert ist, um ein elektromagnetisches Feld zu erzeugen, das durch den ersten Verfolgungssensor (212) der verfolgbaren Nadelanordnung (200), den zweiten Verfolgungssensor des chirurgischen Instruments (140) und das Verfolgungselement (131) des Ultraschallsensors (130) erfasst wird.

5. System zum Durchführen eines perkutanen Navigationsverfahrens nach einem der vorhergehenden Ansprüche, wobei die Rechenvorrichtung (100) konfiguriert ist, um die angezeigte Lage des Ultraschallsensors (130), der verfolgbaren Nadelanordnung (200) und des chirurgischen Instruments (140) in Bezug zueinander zu aktualisieren, während jeweils der Ultraschallsensor, die verfolgbare Nadelanordnung und das chirurgische Instrument bewegt werden.

6. System zum Durchführen eines perkutanen Navigationsverfahrens nach einem der vorhergehenden Ansprüche, wobei die Kamera (210) der verfolgbaren Nadelanordnung (200) mit der verfolgbaren Nadelanordnung einstückig ausgebildet ist.

7. System zum Durchführen eines perkutanen Navigationsverfahrens nach einem der Ansprüche 1 bis 5, wobei die Kamera (210) der verfolgbaren Nadelanordnung (200) mit der verfolgbaren Nadelanordnung abnehmbar verbunden ist.

8. System zum Durchführen eines perkutanen Navigationsverfahrens nach Anspruch 7, wobei die verfolgbare Nadelanordnung (200) mehrere Kameraführungsmerkmale (214) einschließt, die konfiguriert sind, um die Kamera (210) mit der verfolgbaren Nadelanordnung zu verbinden.

## Revendications

1. Système (10) pour réaliser une procédure de navigation percutanée, le système comprenant :
un ensemble aiguille détectable (200) comportant un premier capteur de localisation (212) disposé sur celui-ci et définissant une lumière (206) à travers celui-ci ;
une caméra (210) fixée à l'ensemble aiguille détectable, la caméra étant configurée pour capturer une vidéo ou des images ;
un instrument chirurgical (140) comportant un second capteur de localisation disposé sur celui-ci, l'instrument chirurgical étant conçu pour être inséré à travers la lumière (206) de l'ensemble aiguille détectable (200) ;
un capteur à ultrasons (130) comportant un élément de localisation (131) disposé dessus ; et
un dispositif informatique (100) configuré pour :
suivre un emplacement du capteur à ultrasons (130), de l'ensemble aiguille détectable (200) et de l'instrument chirurgical (140) ; et
afficher l'emplacement du capteur à ultrasons, de l'ensemble aiguille détectable et de l'instrument chirurgical les uns par rapport aux autres.

2. Système pour réaliser une procédure de navigation percutanée selon la revendication 1, dans lequel le dispositif informatique (100) est configuré pour :
recevoir une vidéo ou des images capturées par la caméra (210) ; et
afficher la vidéo ou les images reçues.

3. Système pour réaliser une procédure de navigation percutanée selon la revendication 2, dans lequel la vidéo ou les images affichées de la caméra (210) sont configurées pour être continuellement mises à jour lorsque l'ensemble aiguille détectable (200) est parcouru.

4. Système pour réaliser une procédure de navigation percutanée selon l'une quelconque des revendications précédentes, comprenant en outre un générateur de champ électromagnétique (122) configuré pour générer un champ électromagnétique devant être détecté par le premier capteur de localisation (212) de l'ensemble aiguille détectable (200), le second capteur de localisation de l'instrument chirurgical (140) et l'élément de localisation (131) du capteur à ultrasons (130).

5. Système pour réaliser une procédure de navigation percutanée selon l'une quelconque des revendications précédentes, dans lequel le dispositif informatique (100) est configuré pour mettre à jour l'emplacement affiché du capteur à ultrasons (130), de l'ensemble aiguille détectable (200) et de l'instrument chirurgical (140) l'un par rapport à l'autre lorsque le capteur à ultrasons, l'ensemble aiguille détectable et l'instrument chirurgical sont déplacés.

6. Système pour réaliser une procédure de navigation percutanée selon l'une quelconque des revendications précédentes, dans lequel la caméra (210) de l'ensemble aiguille détectable (200) fait corps avec l'ensemble aiguille détectable.

7. Système pour réaliser une procédure de navigation percutanée selon l'une quelconque des revendications 1 à 5, dans lequel la caméra (210) de l'ensemble aiguille détectable (200) est reliée de manière amovible à l'ensemble aiguille détectable.

8. Système pour réaliser une procédure de navigation percutanée selon la revendication 7, dans lequel l'ensemble aiguille détectable (200) comporte une pluralité de caractéristiques de guidage de caméra (214) configurées pour connecter la caméra (210) à l'ensemble aiguille détectable.
